Europäisches Patentamt

European Patent Office

Office européen des brevets

(19)

(11) Veröffentlichungsnummer: **0 092 718**
**A1**

(12)
# EUROPÄISCHE PATENTANMELDUNG

(21) Anmeldenummer: **83103448.3**

(22) Anmeldetag: **08.04.83**

(51) Int. Cl.³: **G 10 K 11/00**
**A 61 B 10/00**

(30) Priorität: **26.04.82 DE 3215529**

(43) Veröffentlichungstag der Anmeldung:
**02.11.83 Patentblatt 83/44**

(84) Benannte Vertragsstaaten:
**AT CH DE FR GB LI**

(71) Anmelder: **SIEMENS AKTIENGESELLSCHAFT
Berlin und München Wittelsbacherplatz 2
D-8000 München 2(DE)**

(72) Erfinder: **Hetz, Walter
Adam-Kraft-Strasse 17
D-8520 Erlangen(DE)**

(54) Ultraschall-Applikator.

(57) Die Erfindung bezieht sich auf einen Ultraschall-Applikator für die Ultraschallabtastung, insbesondere von körperinternen Organen oder dergleichen, mit einem Ultraschallkopf, der an einem Handgriff gehalten ist. Ziel der Erfindung ist es, einen solchen Ultraschall-Applikator zu schaffen, der insbesondere bei intraoperativem Einsatz immer sicher am Untersuchungsort angekoppelt werden kann. Dieses Ziel wird erfindungs-gemäß dadurch erreicht, daß der Ultraschallkopf (1) mittels Schwenk- oder Drehgelenk (12, 13) an einem Handgriff (14) schwenk- oder drehbar gehaltert ist, der wenigstens im Bereich des Schwenk- oder Drehgelenkes mit einem Klemmschlitz (17) und einer Handhabe (19) zum mehr oder weniger starken Schließen des Klemmschlitzes zum Zwecke der Arretierung des Ultraschallkopfes am Handgriff in einer vorgewählten Schwenk- oder Drehstellung versehen ist.

FIG 1

0092718

SIEMENS AKTIENGESELLSCHAFT     Unser Zeichen

Berlin und München     VPA 82 P 3742 E

## Ultraschall-Applikator

Die Erfindung bezieht sich auf einen Ultraschall-Applikator gemäß den Merkmalen des Oberbegriffes des Patentanspruches 1.

Ein solcher Ultraschall-Applikator soll z. B. auch intra-operativ zur Untersuchung von körperinternen Organen, z. B. Nieren, Leber etc., eingesetzt werden können. Wichtig ist dabei, daß der Ultraschallkopf in der gewünschten Lage immer korrekt am zu untersuchenden Organ angekoppelt ist.

Aufgabe der Erfindung ist es daher, einen solchen Ultraschall-Applikator zu schaffen, der auch bei intra-operativem Einsatz immer sicher am Untersuchungsort angekoppelt werden kann.

Die Aufgabe wird erfindungsgemäß mit den kennzeichnenden Merkmalen des Patentanspruches 1 gelöst.

Ein gemäß der Erfindung ausgebildeter Ultraschall-Applikator erlaubt die sichere Arretierung eines Ultraschallkopfes in einer erwünschten Untersuchungslage am Handgriff. Der Ultraschallkopf läßt sich also immer sicher am Untersuchungsobjekt ankoppeln. Zum Aufsuchen eines geeigneten Applikationsortes kann der Ultraschallkopf bereits in einer bevorzugten Winkelstellung zum Handgriff arretiert sein. Ebenso gut kann der Klemmschlitz aber auch vorher mittels der Handhabe etwas geöffnet werden. Der Ultraschallkopf ist dann am Hand-

Kue 5 Ler / 15.04.1982

griff leicht entriegelt. Er kann dann schwenkbar zum gewünschten Applikationsort geführt und erst dort in der erwünschten Applikationsstellung arretiert werden.

Vorteilhafte Ausgestaltungen der Erfindung ergeben sich aus den Unteransprüchen. Von besonderer Bedeutung sind dabei die Unteransprüche 4, 5 oder 8. Bei Ausbildung des Handgriffes aus Schalen ergibt sich ein vom Ultraschallkopf abnehmbarer Handgriff. Der zerlegte Handgriff läßt sich leichter reinigen und insbesondere vor dem intra-operativen Einsatz problemlos sterilisieren. Als Zusammenhaltglied zum Zusammenhalten der verschiedenen Griffschalen nach Montage am Ultraschallkopf kann dieselbe Handhabe dienen, mit der auch der Klemmschlitz zusammengedrückt wird. Zu diesem Zwecke ist die Handhabe entsprechend Anspruch 6 oder 8 ausgebildet.

Weitere Vorteile und Einzelheiten der Erfindung ergeben sich aus der nachfolgenden Beschreibung von zwei Ausführungsbeispielen anhand der Zeichnung und in Verbindung mit den restlichen Unteransprüchen.

Es zeigen:

Fig. 1 ein erstes Ausführungsbeispiel des erfindungsgemäßen Ultraschall-Applikators in Seitenansicht,

Fig. 2 denselben Ultraschall-Applikator in um 90° gedrehter Ansicht,

Fig. 3 einen Schnitt III-III durch den Schallkopf des Applikators der Figuren 1 und 2,

Fig. 4 ein zweites Ausführungsbeispiel für einen Ultraschall-Applikator gemäß der Erfindung in Seitenansicht,

Fig. 5 denselben Ultraschall-Applikator in Vorderansicht,

Fig. 6 diesen Ultraschall-Applikator in Draufsicht auf den Ultraschallkopf mit abgeschnittenem Haltegriff, und

Fig. 7 Applikationsmöglichkeiten für einen Ultraschall-Applikator bei intra-operativem Einsatz an z. B. einer Niere.

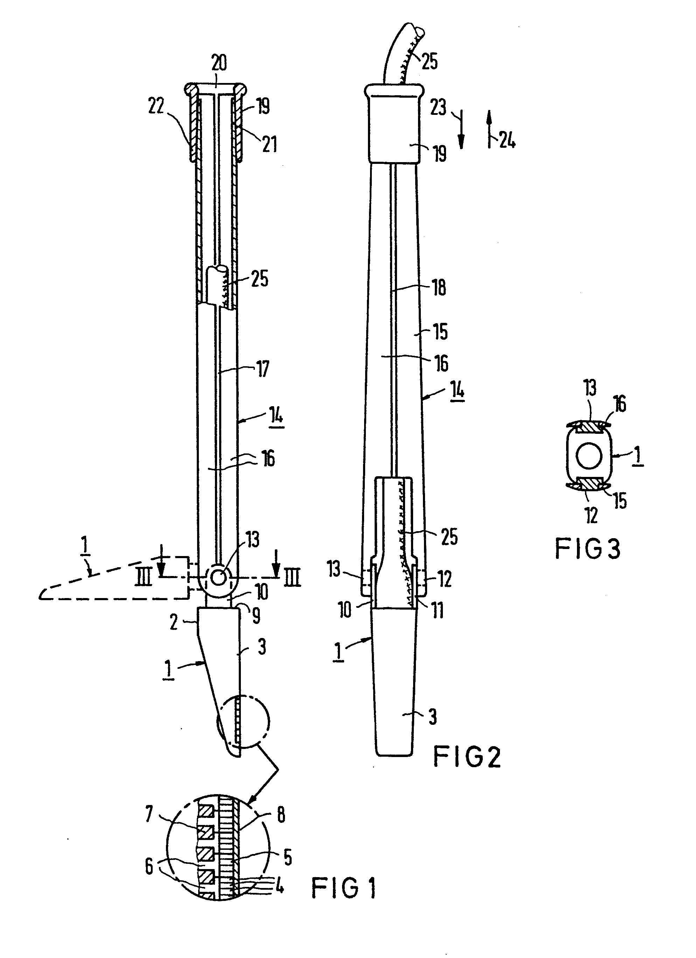

In der Figur 1 ist der Ultraschallkopf ein Ultraschall-
Array 1 mit dem Array-Gehäuse 2 und der Applikationsfläche 3. Das Ultraschall-Array 2 umfaßt dabei an der
Applikationsfläche 3, wie in der Ausschnittsvergrößerung dargestellt ist, eine Vielzahl von Wandlerelementen 4, die im vorliegenden Ausführungsbeispiel feingeteilt sind. Immer mehrere dieser feingeteilten Wandlerelemente 4, z. B. im vorliegenden Fall insgesamt
vier Wandlerelemente, sind elektrisch zu je einer Gruppe 5 (von z. B. insgesamt 48 Gruppen) zusammenkontaktiert. Zur Gruppenkontaktierung dienen Kontaktfahnen 6
eines Kontaktkammes. Mit 7 ist ein Trägerkörper für
die Ultraschall-Wandlerelemente 4 bezeichnet. Der Trägerkörper, in den die Kontaktfahnen 6 eingebettet sind,
besteht beispielsweise aus Epoxydharz mit eingebrachtem oxidiertem Wolframpulver. Mit 8 ist eine Anpassungsschicht für die Wandlerelemente bezeichnet. Die
Anpassungsschicht kann aus Epoxydharz bestehen. Das
Grundprinzip der Feinteilung von Wandlerelementen ist
z. B. in der US-PS 43 05 014 näher beschrieben.

Das Ultraschall-Array 1 umfaßt an seinem hinteren Ende
9 zwei Schenkel 10, 11 (z. B. einer U-Schiene), von
denen jeder, wie in der Figur 2 noch näher dargestellt ist, je einen von zwei Schwenk- oder Drehzapfen 12, 13, die ein Schwenk- oder Drehgelenk bilden, trägt. Ein bis zu den Zapfen 12, 13 geschlitzter Handgriff 14 umgreift federnd mit dem nichtgeschlitzten Ende die Zapfen. Im Ausführungsbeispiel der Figuren 1 bis 3 ist der Handgriff 14 aus zwei Halbschalen 15 und 16 zusammensetzbar, von denen jede geschlitzt ist. In der Seitenansicht der Figur 1 ist der Schlitz der Halbschale 16 mit 17 gekennzeichnet. Die Halbschale 15 besitzt einen entsprechenden Schlitz 17.

Die beiden Halbschalen 15 und 16 sind an den Schwenk- oder Drehzapfen 12, 13 so mit ihren Längskanten auf Abstand montierbar, daß sich entlang den Längskanten wieder je ein Schlitz ergibt. In der Figur 2 ist der vorderseitige Schlitz mit 18 dargestellt. Als Zusammenhaltglied zum Zusammenhalten der beiden Halbschalen 15 und 16 dient eine Kappe 19, die auf das dem Schwenk- oder Drehgelenk 12, 13 abgewandte Ende 20 des Handgriffes 14 aufsetzbar ist. Die Kappe 19 dient dabei gleichzeitig auch zur Arretierung des Ultraschallkopfes 1 in einer gewünschten Winkelstellung zum Handgriff 14. Zu diesem Zwecke verjüngt sich das geschlitzte Griffende 20 zum Ende hin konisch. Diese konische Verjüngung ist in der Figur 1 mit der Kennziffer 21 angedeutet. Die innere Anlagefläche der Kappe 19 verjüngt sich ebenfalls konisch, jedoch gegensinnig zur Verjüngung 21 des Griffendes 20. Die Kappenverjüngung ist mit 22 angedeutet. Der Ultraschall-Applikator 1 wird arretiert, wenn die Kappe in der Pfeilrichtung 23 längs des Haltegriffendes 20 verschoben wird. Hierdurch werden nämlich durch die Keilwirkung der Kappe 19 die geschlitzten Enden 20 des Haltegriffes 14 zusammengedrückt. Der Preßdruck überträgt sich über die Halbschalen 15 und 16 auf die

- 5 -     VPA  82 P 3742  E

Schwenk- oder Drehzapfen 12 und 13. Die Halbschalen werden an den Schwenk- oder Drehzapfen angeklemmt und der Ultraschall-Applikator ist dadurch arretiert. Eine Entriegelung der Arretierung wird dadurch erreicht, daß die Kappe 19 in Pfeilrichtung 24 zurückgeschoben wird. Damit lockert sich der Druck auf die geschlitzten Enden 20 des Haltegriffes 14. Entsprechend wird kein oder nur wenig Preßdruck auf die Zapfen 12, 13 übertragen. Das Ultraschall-Array kann also relativ zum Haltegriff geschwenkt werden. Im Ausführungsbeispiel der Figuren 1 bis 3 ist das Signalanschlußkabel 25 des Ultraschall-Arrays 1 im Haltegriff 14 geführt.

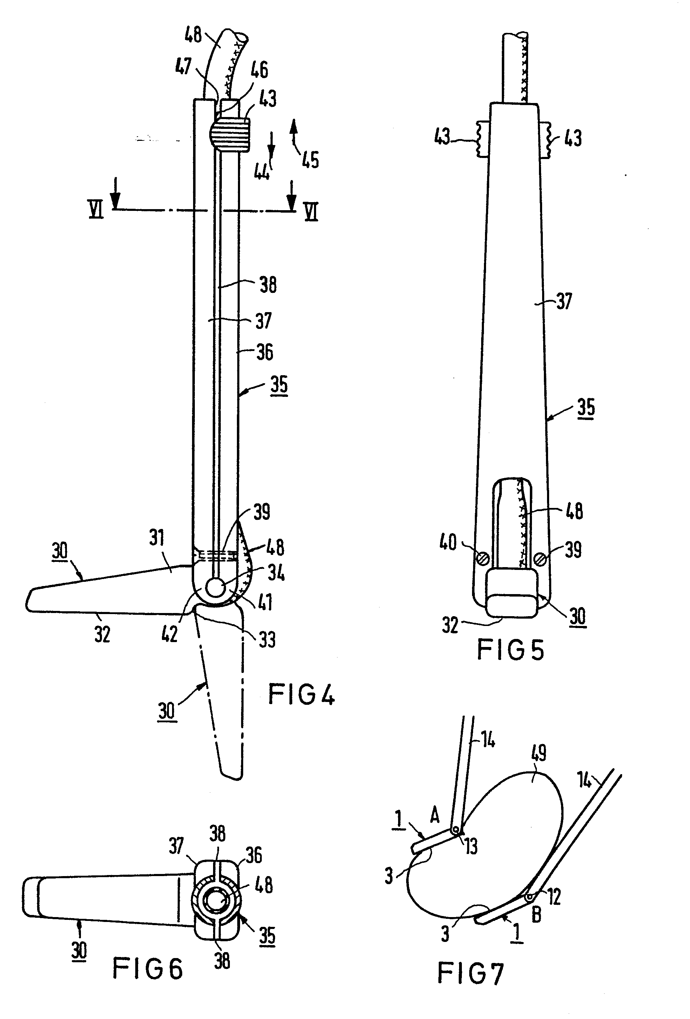

Ein weiteres Ausführungsbeispiel zeigen die Figuren 4 bis 6. Hier ist das Ultraschall-Array mit 30 bezeichnet. Es umfaßt das Array-Gehäuse 31 mit der Applikationsfläche 32. Ansonsten ist das Array genauso aufgebaut wie jenes der Figuren 1 bis 3. Es umfaßt am hinteren Ende 33 also vorzugsweise wieder zwei (hier jedoch im einzelnen nicht näher dargestellte) Schenkel, die wieder ein Schwenk- oder Drehgelenk bildende Schwenk- oder Drehzapfen umfassen, von denen in der Figur 4 jedoch nur einer mit der Kennziffer 34 dargestellt ist. Der Handgriff 35 besteht wieder aus zwei Halbschalten 36 und 37. Im Gegensatz zum Ausführungsbeispiel der Figuren 1 bis 3 sind diese Halbschalen jedoch nicht extra geschlitzt. Der elastische Schlitz, der in der Figur 4 mit 38 gekennzeichnet ist, ergibt sich vielmehr dadurch, daß die beiden Halbschalen 36, 37 mittels Schrauben 39 und 40 an den Schwenk- oder Drehzapfen 34 in der Weise zum Handgriff montiert sind, daß die Spalte zwischen den auf Abstand parallel verlaufenden Längskanten der Halbschalen allein die Handgriffschlitze bilden. Bei dieser Spezialausbildung umgreift also der Handgriff 35 mit den unteren Enden der jeweiligen Halbschale jeden der beiden Schwenk- oder
Drehzapfen 34 nach Art einer Zange, was in der Figur 4
für den rechtsseitigen Schwenk- oder Drehzapfen 34 mit
den Zangenbacken 41 und 42 angedeutet ist. Der Zangendrehpunkt der Zange wird dabei durch die Position der
Schrauben 39 und 40 auf der den Schlitzenden gegenüberliegenden Seite der Schwenk- oder Drehzapfen festgelegt. Als Handhabe zum Lockern oder Schließen der
Zange dient ein Spreizglied 43, das in Längsrichtung
des Schlitzes 38 des Handgriffes 35 bewegbar ist. Wird
das Spreizglied 43 in Richtung des Pfeiles 44 bewegt,
so werden die beiden Halbschalen des Handgriffes auseinandergespreizt. Die Backen 41 und 42 der Halbschalen drücken auf die Zapfen 34 und das Ultraschall-
Array wird dadurch in vorgegebener Winkelposition zum
Haltegriff arretiert. Wird das Spreizglied 43 in Gegenpfeilrichtung 45 wieder in die dargestellte Ausgangsposition zurückgeschoben (das Spreizglied 43 rastet
dann in eine Ausnehmung 46 am oberen geschlitzten Ende
47 des Haltegriffes 35), so sind die Halbschalen wieder ungespreizt; der Preßdruck der Zangen 41 und 42 auf
die Schwenk- und Drehachsen 34 lockert sich und das
Ultraschall-Array 30 kann dann relativ zum Haltegriff
bewegt werden. Auch im Ausführungsbeispiel der Figuren
4 bis 6 wird das Signalschlußkabel 48 für das Ultraschall-Array 30 in der dargestellten Weise im Haltegriff 35 geführt.

In beiden Ausführungsbeispielen sind die Handgriff-
Halbschalen vorzugsweise aus rostfreiem Stahl gefertigt. Halbschalen aus diesem Material lassen sich
leicht reinigen und insbesondere vor einem intra-operativen Einsatz problemlos sterilisieren. Reinigung und
Sterilisation werden an den Halbschalen bei zerlegtem
Handgriff vorgenommen. Das Array-Gehäuse kann ebenfalls

0092718

aus rostfreiem Stahl oder aus einem anderen nicht rostenden Material, z. B. auch aus einem Kunststoff, bestehen. Vor Gebrauch wird über Array und Handgriff eine sterilisierte Isolierkappe, z. B. eine Gummikappe, gezogen.

Die Ultraschall-Applikatoren der Figuren 1 bis 6 eignen sich insbesondere für den intra-operativen Einsatz, obgleich auch ein subkutaner Einsatz möglich ist. Die Möglichkeit des intra-operativen Einsatzes ist am Beispiel einer Nierenuntersuchung in der Figur 7 dargestellt. Hier ist die durch die Operation freigelegte Niere mit 49 bezeichnet. Das Ultraschall-Array liegt in der Untersuchungsposition A  z. B. auf der Vorderwand der Niere. In der Untersuchungsposition B liegt das Ultraschall-Array hingegen an der Rückwand der Niere. Es ist selbstverständlich, daß derartige Untersuchungen nicht nur an der Niere, sondern an jedem beliebigen anderen körperinternen Organ oder einem sonstigen inneren Körperteil vorgenommen werden können.

8 Patentansprüche
7 Figuren

Patentansprüche

1. Ultraschall-Applikator für die Ultraschallabtastung, insbesondere von körperinternen Organen oder dergleichen, mit einem Ultraschallkopf, der an einem Handgriff gehaltert ist, d a d u r c h  g e k e n n - z e i c h n e t , daß der Ultraschallkopf (1; 30) mittels Schwenk- oder Drehgelenk (12, 13; 34) an einem solchen Handgriff (14; 35) schwenk- oder drehbar gehaltert ist, der wenigstens im Bereich des Schwenk- oder Drehgelenkes mit einem Klemmschlitz (17; 38) und einer Handhabe (19; 43) zum mehr oder weniger starken Schließen des Klemmschlitzes zum Zwecke der Arretierung des Ultraschallkopfes am Handgriff in einer vorgewählten Schwenk- oder Drehstellung versehen ist.

2. Ultraschall-Applikator nach Anspruch 1, d a - d u r c h  g e k e n n z e i c h n e t , daß ein bis zum Schwenk- oder Drehgelenk (12, 13) geschlitzter Handgriff (14) mit dem nicht geschlitzten Ende das Schwenk- oder Drehgelenk (12, 13) am Ultraschallkopf (1) federnd umgreift und daß die Handhabe ein Zusammendrückelement (19) für den geschlitzten Handgriff ist (Figuren 1 bis 3).

3. Ultraschall-Applikator nach Anspruch 2, d a - d u r c h  g e k e n n z e i c h n e t , daß der geschlitzte Handgriff (14) an dem dem Schwenk- oder Drehgelenk (12, 13) abgewandten geschlitzten Griffende (20) sich zum Ende hin konisch verjüngt und daß das Zusammendrückelement (19) eine sich gegensinnig konisch verjüngende Kappe ist, die auf dem sich konisch verjüngenden Ende (21) des geschlitzten Handgriffes in Konuslängsrichtung verschiebbar ist und je nach Verschieberichtung (23, 24) durch Einwirkung auf das ge-

0092718

schlitzte Handgriffende den Schlitz (17) des Handgriffes mehr oder weniger stark öffnet oder zusammendrückt.

4. Ultraschall-Applikator nach Anspruch 2 oder 3,
d a d u r c h   g e k e n n z e i c h n e t ,   daß
der Handgriff (14) aus einer vorgebbaren Zahl von geschlitzten Schalen (15, 16) zusammengesetzt ist, die
jederzeit wieder lösbar am Schwenk- oder Drehgelenk
(12, 13) des Ultraschallkopfes (1) montiert sind.

5. Ultraschall-Applikator nach den Ansprüchen 3 und 4,
d a d u r c h   g e k e n n z e i c h n e t ,   daß
der Handgriff (14) aus zwei Halbschalen (15, 16) zusammensetzbar ist, von denen vorzugsweise jede bis
zum Schwenk- oder Drehgelenk (12, 13) des Ultraschallkopfes (1) geschlitzt ist und mit dem nicht geschlitzten Ende das Schwenk- oder Drehgelenk umgreift und von
denen jede an dem dem Schwenk- oder Drehgelenk abgewandten geschlitzten Halbschalenende (20) sich konisch
verjüngt (21) und daß als Zusammenhaltglied zum Zusammenhalten der beiden Halbschalen (15, 16) nach Montage
am Ultraschallkopf (1) die Kappe (19) dient, die auch
zur Arretierung des Schwenk- oder Drehgelenkes aufgrund
Zusammendrückens der Klemmschlitze benutzt wird.

6. Ultraschall-Applikator nach Anspruch 1,   d a -
d u r c h   g e k e n n z e i c h n e t ,   daß ein
geschlitzter Handgriff (35) im Bereich des Schwenk-
oder Drehgelenkes (34) mit seinen geschlitzten Enden
(41, 42) das Schwenk- oder Drehgelenk nach Art einer
Zange, deren Zangendrehpunkt (39, 40) auf der den
Schlitzenden gegenüberliegenden Seite des Schwenk-
oder Drehgelenkes liegt, umgreift, und daß dem geschlitzten Handgriff (35) eine Handhabe (43) zum Lokkern oder Schließen der Zange zugeordnet ist (Figuren
4 bis 6).

**0092718**

7. Ultraschall-Applikator nach Anspruch 6,    d a - d u r c h   g e k e n n z e i c h n e t ,    daß die Handhabe (43) ein in Längsrichtung des Schlitzes (38) des Handgriffes (35) bewegliches Spreizglied ist.

8. Ultraschall-Applikator nach Anspruch 6 oder 7, d a d u r c h   g e k e n n z e i c h n e t ,    daß der Handgriff (35) aus wenigstens zwei Halbschalen (36, 37) besteht, die mittels Schraubverbindung (39, 40) oder dergleichen am Schwenk- oder Drehgelenk (34) des Ultraschallkopfes (30) zum Handgriff montierbar sind, in der Weise, daß die auf Abstand parallel verlaufenden Längskanten der Halbschalten wenigstens einen, vorzugsweise jedoch zwei, Schlitze (38) im Handgriff bilden und die Schraubverbindung (39, 40) den Zangendrehpunkt definiert.

FIG 1

FIG 2

FIG 3

0092718

FIG 4

FIG 5

FIG 6

FIG 7

**0092718**

Nummer der Anmeldung

**EUROPÄISCHER RECHERCHENBERICHT**

Europäisches
Patentamt

EP 83 10 3448

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | EP-A-0 039 045 (OLYMPUS OPTICAL CO., LTD.)<br>* Seite 2, Zeile 34 - Seite 4, Zeile 14; Seite 8, Zeile 2 - Seite 9, Zeile 12; Abbildungen 2,3,5-7 * | 1 | G 10 K 11/00<br>A 61 B 10/00 |
| | --- | | |
| Y | O. RICHTER und R.v. VOSS: "Bauelemente der Feinmechanik", 9. Auflage, 1959, Seite 282, VEB Verlag Technik Berlin, Berlin, DE.<br>* Seite 282, Absätze 1,2; Abbildung 42.84 * | 1-3 | |
| | --- | | |
| A | EP-A-0 039 851 (OLYMPUS OPTICAL CO., LTD.)<br>* Seite 3, Zeile 21 - Seite 5, Zeile 5; Abbildungen 2-6 * | 1 | |
| | --- | | |
| A | FR-A-2 474 303 (BRICE M.A.)<br><br>* Seite 5, Zeilen 15-19; Seite 10, Zeilen 5-8; Abbildungen 1,2 * | 1,2,4 5 | |
| | --- | | |
| A | US-A-4 174 715 (HASSAN)<br><br>* Spalte 4, Zeile 66 - Spalte 5, Zeile 13; Abbildungen 7-13 * | | |
| | --- | | |
| A | DE-A-2 735 722 (SIEMENS AG) | | |
| | ---     -/- | | |

| RECHERCHIERTE SACHGEBIETE (Int. Cl. 3) |
|---|
| A 61 B 10/00<br>A 61 B 17/28<br>A 61 B 19/00<br>G 01 N 29/00<br>G 10 K 11/00<br>G 10 K 11/34 |

Der vorliegende Recherchenbericht wurde für alle Patentansprüche erstellt.

| Recherchenort | Abschlußdatum der Recherche | Prüfer |
|---|---|---|
| DEN HAAG | 02-08-1983 | RIEB D.K. |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | FR-A-1 514 314 (ALVAR ELECTRONIC) | | |

RECHERCHIERTE SACHGEBIETE (Int. Cl. ³)

Der vorliegende Recherchenbericht wurde für alle Patentansprüche erstellt.

| Recherchenort DEN HAAG | Abschlußdatum der Recherche 02-08-1983 | Prüfer RIEB D.K. |
|---|---|---|